# EUROPEAN PATENT APPLICATION

(11) **EP 4 682 329 A1**
(43) Date of publication of application: **21.01.2026**
(21) Application number: 25188963.0
(22) Date of filing: 11.07.2025
(51) Int. Cl.: E04H 7/20, E04H 7/06, E04H 7/26, A01C 3/02, C12M 1/107

(54) **CONCRETE TANK FOR FERMENTATION WITH OPTIMIZED ROOF CONSTRUCTION, METHOD FOR MANUFACTURING AND USE OF SUCH CONCRETE TANK FOR FERMENTATION**

(30) Priority: 12.07.2024 BE 202405458
(71) Applicant: Bio-Dynamics nv, 8710 Wielsbeke (BE)
(72) Inventor: VAN DAMME, Thomas, 8710 Wielsbeke (BE)
(74) Representative: Brantsandpatents bv

(57) **Abstract**

The present invention relates to a concrete tank for fermentation comprising a concrete floor slab, a cylindrical concrete side wall, and a concrete roof structure, wherein the concrete roof structure is a self-supporting concrete structure, wherein the concrete roof structure is post-tensioned by means of a plurality of metal tendons in the concrete roof structure, wherein the metal tendons form chords of a circle, wherein the circle is concentric with a circular cross-section of the cylindrical concrete side wall and wherein the metal tendons, as viewed in a height direction, are located in a lower half of the concrete roof structure. The invention also relates to a method for manufacturing a concrete tank for fermentation and to the use of such a concrete tank for fermentation of biomass.

## Description

### TECHNICAL FIELD

The invention relates to a concrete tank, more specifically a concrete tank for fermentation. In a second aspect, the invention also relates to a method for manufacturing such a concrete tank. In another aspect, the invention also relates to a use of the concrete tank for biomass fermentation.

### PRIOR ART

Concrete tanks as fermentation tanks are known from the prior art. Such a tank has a concrete floor slab, a cylindrical concrete side wall and a concrete roof structure. Biomass is added to the concrete tank. An example of a suitable biomass is manure. The biomass ferments in the tank, wherein digestate and mainly methane gas is formed. This methane gas is usually used in a cogeneration plant for generating heat and electricity or purified and pumped into the gas network.

The formation of gas creates an overpressure in the concrete tank, for example, an overpressure of 50 mbar. This overpressure must be absorbed by the cylindrical side wall and the concrete roof structure. The cylindrical wall must also withstand the pressure of the biomass on the wall. These pressure forces are much greater than the overpressure from the formed gas, so the cylindrical wall can normally withstand the overpressure without any problem.

The concrete roof structure is preferably constructed as lightly as possible. The concrete roof structure namely rests on the concrete side wall, so the heavier the concrete roof structure, the stronger the concrete side wall must be. The concrete roof structure will also sag under its own weight, causing tensile forces to occur on a bottom surface of the concrete roof structure, which cause cracks. Additionally, it is also common to install an agitator in the concrete tank, which is suspended from the concrete roof structure. The agitator serves to stir the biomass so that the fermentation proceeds evenly and stably. The agitator has such a weight that it again causes tensile forces in the concrete roof structure, which again results in a small amount of crack formation. The concrete roof structure will heat up and cool down due to environmental factors, causing the concrete to expand and contract. This is again a cause of minor crack formation. External loads such as snow, wind, technical infrastructure on the concrete roof structure, and the like further contribute to the crack formation. A concrete roof structure is usually made of reinforced concrete. Reinforcement meshes are installed in the concrete of the roof structure. The concrete roof structure is thus constructed strong enough not to break, but the small cracks form gas leaks, allowing formed gas to escape into the environment. To avoid gas leaks, a lining is often applied to the inside of the concrete tank to make the concrete tank gas-tight. The disadvantage of such lining is that it has a short lifespan, sometimes only ten years, requiring the lining to be repaired multiple times during the lifespan of the concrete tank. Repairs may require immediate repair in case of an acute leak, causing the concrete tank to be emptied unexpectedly.

Another disadvantage of known concrete tanks is that the concrete roof structure is usually constructed in a dome shape. This makes the installation of further technical infrastructure, such as pumps, motors, and the like, on the concrete roof structure complex. With a dome-shaped roof, it is also necessary to place walkways on top of the concrete tank in order to safely access the technical infrastructure.

The present invention aims to provide at least a solution for some of the above-mentioned problems or disadvantages.

### SUMMARY OF THE INVENTION

In a first aspect, the present invention relates to a device according to claim 1.

The self-supporting concrete structure is advantageous because it eliminates the need for heavy concrete crossbeams in the concrete tank to support the concrete roof structure. This increases the internal volume of the concrete tank. Because the concrete roof structure is post-tensioned by means of the multiple metal tendons and because the metal tendons are located in a lower half of the concrete roof structure, the concrete roof structure is in effect lifted. This allows the concrete roof structure to be made both self-supporting and lighter. It is therefore also possible to realize the concrete roof structure as flat or with only a very slight slope, which greatly simplifies the placement of technical infrastructure on the roof, and whereby it is not necessary to place walkways on the concrete roof structure to safely access this technical infrastructure. It is also advantageous that because the concrete roof structure is lifted, cracks in the concrete roof structure are avoided as much as possible and it remains gas-tight. The fact that the concrete roof structure remains gas-tight is particularly beneficial because the concrete roof structure has a very long lifespan of up to fifty years or more. Even when using a lining as an additional gas-tight barrier, a defect in the lining does not pose an immediate problem and a repair or renovation of the lining can be scheduled.

Preferred embodiments of the device are set out in claims 2-9.

A specific preferred embodiment concerns a device according to claim 4.

The concrete roof structure is subject to a first bending moment. The first bending moment is created by forces acting on the concrete roof structure, such as the dead weight of the concrete roof structure, the weight of an optional agitator, the weight of technical infrastructure on the concrete roof structure, and external loads such as snow, wind, etc. The first bending moment equals the magnitude of the force multiplied by the perpendicular distance from a point where the force acts to a point where the first bending moment acts. The first bending moment causes tensile and compressive stresses in the concrete roof structure. The metal tendons are post-tensioned over their entire length with the same post-tensioning force. The post-tensioning force generates a second bending moment equal to the magnitude of the post-tensioning force multiplied by the perpendicular distance to the centroid of the concrete roof structure. This second bending moment partially or completely compensates for the first bending moment. The first bending moment is greatest towards the center of the concrete roof structure. Because the post-tensioning force remains the same over the entire length of a metal tendon, a greater perpendicular distance between the post-tensioning force and the centroid is needed in the center to be able to compensate for the first bending moment with the second bending moment, than at an edge of the concrete roof structure. In practice, a greater distance is thus needed between the metal tendons and the centroid in the center than at the edge of the concrete roof structure. Because the concrete roof structure has a decreasing height from the center of the circle to the edge, the metal tendons are located further from the centroid towards the center of the circle than at the edge, and less concrete can also be used for the entire concrete roof structure. This results in an even lighter concrete roof structure.

In a second aspect, the present invention relates to a method according to claim 10.

This method has the advantage, inter alia, that a concrete tank with a self-supporting and lighter concrete roof structure can be constructed with only a limited number of steps. It is additionally advantageous that the method also makes it possible to create the concrete roof structure flat or with only a very slight angle of inclination, which greatly simplifies the placement of technical infrastructure on the roof and makes walkways on the concrete roof structure superfluous. It is also advantageous that by carrying out the method, a concrete tank is obtained, wherein cracks in the concrete roof structure are avoided as much as possible, whereby the concrete roof structure remains gas-tight.

Preferred embodiments of the method are described in dependent claims 11-14.

In a third aspect, the present invention relates to a use according to claim 15.

This use results in advantageous pre-acidification (hydrolysis) and/or fermentation of biomass in a concrete tank, wherein because of the concrete roof structure of the concrete tank, no or only very minimal methane gas escapes from the concrete tank. Methane gas is harmful to the climate. By avoiding cracks in the concrete roof structure, a higher gas yield from the fermentation can also be obtained.

### DESCRIPTION OF THE FIGURES

**Figure 1** shows a top view of a concrete tank according to an embodiment of the present invention.
**Figure 2** shows a cross-section in a vertical plane of a concrete tank according to an embodiment of the present invention.
**Figure 3** shows a detailed cross-section in a vertical plane of a concrete tank according to an embodiment of the present invention.

### DETAILED DESCRIPTION

Unless otherwise defined, all terms used in the description of the invention, including technical and scientific terms, have the meanings as commonly understood by a person skilled in the art to which the invention pertains. For a better understanding of the description of the invention, the following terms are explicitly explained.

In this document, "a" and "the" refer to both the singular and the plural, unless the context presupposes otherwise. For example, "a segment" means one or more segments.

The terms "comprise," "comprising," "consist of," "consisting of," "provided with," "include," "including," "contain," "containing," are synonyms and are inclusive or open terms that indicate the presence of what follows, and which do not exclude or prevent the presence of other components, characteristics, elements, members, steps, as known from or disclosed in the prior art.

Quoting numerical ranges by endpoints includes all integers, fractions and/or real numbers between the endpoints, these endpoints included.

In the context of this document, a centerline is a central reference line that runs through the middle of an object.

In the context of this document, a self-supporting concrete structure is a structural element that can independently bear a load without additional support from other structural elements. This means that the structural element is capable of supporting its own weight and additional loads without the help of crossbeams.

In the context of this document, a chord is a line that connects two points on a circumference of a circle.

In the context of this document, grout is a material that is used to fill and reinforce spaces. It comprises a mixture of cement, water, sand, and sometimes fine gravel or other fillers. Grout is applied in liquid or paste-like form and hardens into a strong, durable mass.

In a first aspect, the invention relates to a concrete tank for fermentation.

The concrete tank comprises a concrete floor slab, a cylindrical concrete side wall, and a concrete roof structure.

The concrete floor slab is preferably a disk-shaped floor slab. The concrete floor slab is preferably reinforced using reinforcement meshes. The reinforcement meshes preferably have a mesh size of at most 150 mm, more preferably at most 100 mm.

The bars of the reinforcement meshes preferably have a diameter of at least 10 mm, more preferably at least 12 mm. Preferably, the floor slab comprises an upper reinforcement and a lower reinforcement. The upper reinforcement is preferably at a distance of at least 30 mm from a top surface of the concrete floor slab, more preferably at least 40 mm. The upper reinforcement is preferably at a distance of at most 70 mm from the top surface of the concrete floor slab, more preferably at most 60 mm, even more preferably at most 55 mm, and even more preferably at most 50 mm. Bars of reinforcement meshes of the upper reinforcement preferably have a diameter of at least 14 mm, more preferably at least 16 mm. The lower reinforcement is preferably at a distance of at least 30 mm from a bottom surface of the concrete floor slab, more preferably at least 40 mm. The lower reinforcement is preferably at a distance of at most 70 mm from the bottom surface of the concrete floor slab, more preferably at most 60 mm, even more preferably at most 55 mm, and even more preferably at most 50 mm. The concrete floor slab is placed directly on a ground surface or on a foundation. The foundation is a trench or pile foundation. Preferably, the foundation is a pile foundation.

The concrete cylindrical side wall is preferably cast in place. The concrete cylindrical side wall is preferably cast layer by layer. The concrete side wall is preferably reinforced. The concrete side wall is reinforced using reinforcement meshes. Alternatively, the concrete side wall is reinforced with pre-tensioned or post-tensioned tendons, wherein the pre-tensioned or post-tensioned tendons are placed in circular loops in the concrete cylindrical side wall. The cylindrical loops are concentric with the circumference of the concrete cylindrical side wall. Alternatively, the concrete cylindrical side wall is reinforced with a combination of at least two elements from a group consisting of reinforcement meshes, pre-tensioned tendons, and post-tensioned tendons. Preferably, the concrete cylindrical side wall is reinforced at least by means of post-tensioned tendons. The concrete cylindrical side wall is preferably insulated. This is advantageous for stable conditions in the concrete tank for fermentation. The concrete cylindrical side wall is optionally heated by means of pipes in the cylindrical side wall.

The concrete roof structure is preferably reinforced. The concrete roof structure is preferably reinforced using reinforcement meshes. The reinforcement meshes preferably have a mesh size of at most 150 mm. The bars of the reinforcement meshes preferably have a diameter of at least 6 mm, more preferably at least 8 mm. Preferably, the concrete roof structure comprises an upper reinforcement and a lower reinforcement. The upper reinforcement is preferably at a distance of at least 30 mm from a top surface of the concrete roof structure, more preferably at least 40 mm. The upper reinforcement is preferably at a distance of at most 70 mm from the top surface of the concrete roof structure, more preferably at most 60 mm, even more preferably at most 55 mm, and even more preferably at most 50 mm. The lower reinforcement is preferably at a distance of at least 30 mm from a bottom surface of the concrete roof structure, more preferably at least 40 mm. The lower reinforcement is preferably at a distance of at most 70 mm from the bottom surface of the concrete roof structure, more preferably at most 60 mm, even more preferably at most 55 mm, and even more preferably at most 50 mm.

The concrete roof structure is a cast-in-place roof structure. For this, a temporary scaffolding is built inside the concrete tank to support a formwork for casting the concrete roof structure.

Optionally, the concrete tank includes an agitator suspended from the concrete roof structure. In that case, the concrete roof structure preferably comprises a central opening for lowering the agitator into the concrete tank.

According to a preferred embodiment, the concrete roof structure is a self-supporting concrete structure. The concrete roof structure is post-tensioned by means of multiple metal tendons in the concrete roof structure. By post-tensioned is meant that the metal tendons are tensioned only after the casting and curing of the concrete and not before the casting of the concrete as with pre-tensioned tendons. The metal tendons are preferably steel tendons. The metal tendons form chords of a circle. The circle is concentric with a circular cross-section of the cylindrical concrete side wall. It is clear that the cylindrical side wall has a cylinder axis and that the circular cross-section lies in a plane transverse to the cylinder axis. Being concentric means that after perpendicular projection of the circle onto the said plane, the circle and circular cross-section are concentric. The circle and the circular cross-section thus do not have to lie in the same plane. The metal tendons are, viewed in a height direction, located in a lower half of the concrete roof structure. This means that, viewed in a vertical cross-section of the concrete roof structure, the metal tendons are located lower than a centerline of the concrete roof structure in the said vertical cross-section. The lower part of the concrete roof structure is the part that comes under tension due to its own weight, which can cause small cracks to form in the lower part.

It is clear that in addition to the metal tendons that form the chords, other metal tendons, whether or not pre-tensioned or post-tensioned, can be placed in the concrete roof structure. In the context of this document, "metal tendons" refers to the metal tendons that form a chord, unless explicitly stated otherwise.

Each metal tendon is slidably placed in a sheath. The sheath is preferably a plastic sheath that is placed in a formwork for the concrete roof structure before casting the concrete and which is cast into the concrete. The sheath is advantageous because, as a result, the metal tendon is not bonded to the concrete and can be tensioned after the concrete has cured. The metal tendon is preferably greased to facilitate the sliding of the metal tendon in the sheath. Each metal tendon is attached at a first end to a fixed point in the concrete roof structure. For reasons of practical implementation, it is possible for the fixed point to be located outside the circle. It is clear that in that case the metal tendon extends beyond the circle to the fixed point. At a point of a second, opposite end of the metal tendon, an additional formwork is placed, into which no concrete is initially cast. An anchor is placed at the point of the second end of the metal tendon. The anchor is suitable for post-tensioning the metal tendon. Such anchors are known from the prior art. Because no concrete is initially cast at the point of the second end of the metal tendon, the anchor is accessible and the metal tendon can be tensioned after the concrete has cured. The anchor is accessible, for example, from a top side, an underside, or a side of the concrete roof structure. For reasons of practical implementation, it is possible for the anchor to be located outside the circle. It is clear that in that case the metal tendon extends beyond the circle into the anchor. The anchor is preferably encased in concrete after the post-tensioning of the metal tendon, so that the anchor is firmly integrated into the concrete roof structure. This protects the anchor and the metal tendon against corrosion. The sheaths are not filled. This form of post-tensioning is known as unbonded post-tensioning.

Alternatively, each metal tendon is slidably placed in a duct in the concrete roof structure. Each metal tendon is attached at a first end to a fixed point in the concrete roof structure. For reasons of practical implementation, it is possible for the fixed point to be located outside the circle. It is clear that in that case the metal tendon extends beyond the circle to the fixed point. At a point of a second, opposite end of the metal tendon, an additional formwork is placed, into which no concrete is initially cast. At the point of the second end of the metal tendon, an anchor for post-tensioning the metal tendon is placed. The anchor is accessible, for example, from a top side, an underside, or a side of the concrete roof structure. For reasons of practical implementation, it is possible for the anchor to be located outside the circle. It is clear that in that case the metal tendon extends beyond the circle into the anchor. After the concrete has cured, the metal tendon is tensioned. The ducts in the concrete roof structure are filled with grout after the tensioning of each metal tendon. The anchor is preferably encased in concrete after the post-tensioning of the metal tendon, so that the anchor is firmly integrated into the concrete roof structure. This protects the anchor and the metal tendon against corrosion. This form of post-tensioning is known as bonded post-tensioning.

The self-supporting concrete structure is advantageous because it eliminates the need for heavy concrete crossbeams in the concrete tank to support the concrete roof structure. This increases the internal volume of the concrete tank. Because the concrete roof structure is post-tensioned by means of the multiple metal tendons and because the metal tendons are located in a lower half of the concrete roof structure, the concrete roof structure is in effect lifted. This allows the concrete roof structure to be made both self-supporting and lighter. It is therefore also possible to realize the concrete roof structure as flat or with only a very slight slope, which greatly simplifies the placement of technical infrastructure on the roof, and whereby it is not necessary to place walkways on the concrete roof structure to safely access this technical infrastructure. It is also advantageous that, because the concrete roof structure is lifted up, tension in the lower half of the concrete roof structure is minimized as much as possible, whereby cracks in the concrete roof structure are avoided as much as possible and it remains gas-tight. The fact that the concrete roof structure remains gas-tight is particularly beneficial because the concrete roof structure has a very long lifespan of up to fifty years or more. Even when using a lining as an additional gas-tight barrier, a defect in the lining does not pose an immediate problem and a repair or renovation of the lining can be scheduled.

According to one embodiment, the circle has a radius that is equal to or greater than 0.95 times a radius of the cylindrical concrete side wall, wherein the radius of the cylindrical concrete side wall is measured on an interior side of the concrete tank. Preferably, the radius of the circle is equal to or greater than 0.96 times the radius of the cylindrical concrete side wall, more preferably equal to or greater than 0.97 times the radius of the cylindrical concrete side wall, even more preferably equal to or greater than 0.98 times the radius of the cylindrical concrete side wall, even more preferably equal to or greater than 0.99 times the radius of the cylindrical concrete side wall, and even more preferably equal to or greater than the radius of the cylindrical concrete side wall. Most preferably, the radius of the circle is equal to a radius of an outer circumference of the concrete tank. The radius of the outer circumference is measured on an outer side of the cylindrical concrete side wall. This embodiment is advantageous because it allows the concrete roof structure to be lifted up by the metal tendons to a point where the concrete roof structure is supported by the cylindrical concrete side wall.

According to a preferred embodiment, at least a portion of the metal tendons form a grid pattern. The grid pattern is preferably a rectangular grid pattern. Preferably, an opening in the grid has sides with a maximum dimension of 70 cm, more preferably 60 cm, even more preferably 50 cm, and even more preferably 40 cm. The grid pattern is formed by the crossing of the metal tendons. The metal tendons cross each other at a distance from a center point of the circle that is preferably less than or equal to 60% of a radius of the circle, more preferably less than or equal to 50%, even more preferably less than or equal to 40%, and even more preferably less than or equal to 35%. This embodiment is advantageous because it results in the greatest possible concentration of post-tensioned tendons near the center point of the circle, where the concrete roof structure is the heaviest and the stresses in the concrete roof structure are the highest.

According to a preferred embodiment, at least a portion of the tendons are radii extending from a center point of the circle. A radius is formed by one or more metal tendons. The concrete roof structure comprises multiple radii formed by one or more tendons. Preferably, the radii are evenly distributed around the center of the concrete roof structure. Preferably, there is an angle of at most 20° between two radii, more preferably at most 15°, even more preferably at most 10°, and even more preferably at most 5°. This embodiment is advantageous because it results in the greatest possible concentration of post-tensioned tendons near the center point of the circle, where the concrete roof structure is the heaviest and the stresses in the concrete roof structure are the highest.

This embodiment can advantageously be combined with a previously described embodiment, wherein at least a portion of the metal tendons form a grid pattern.

According to a preferred embodiment, the concrete roof structure, when viewed in a vertical plane through a center point of the circle, has a cross-section that has a decreasing height from the center point of the circle toward an edge of the concrete roof structure. The height can decrease stepwise or continuously. Preferably, the height decreases continuously.

The concrete roof structure is subject to a first bending moment. The first bending moment is created by forces acting on the concrete roof structure, such as the dead weight of the concrete roof structure, the weight of an optional agitator, the weight of technical infrastructure on the concrete roof structure, and external loads such as snow, wind, etc. The first bending moment equals the magnitude of the force multiplied by the perpendicular distance from a point where the force acts to a point where the first bending moment acts. The first bending moment causes tensile and compressive stresses in the concrete roof structure. The metal tendons are post-tensioned over their entire length with the same post-tensioning force. The post-tensioning force generates a second bending moment equal to the magnitude of the post-tensioning force multiplied by the perpendicular distance to the centroid of the concrete roof structure. This second bending moment partially or completely compensates for the first bending moment. The first bending moment is greatest towards the center of the concrete roof structure. Because the post-tensioning force remains the same over the entire length of a metal tendon, a greater perpendicular distance between the post-tensioning force and the centroid is needed in the center to be able to compensate for the first bending moment with the second bending moment, than at an edge of the concrete roof structure. In practice, a greater distance is thus needed between the metal tendons and the centroid in the center than at the edge of the concrete roof structure. Because the concrete roof structure has a decreasing height from the center of the circle to the edge, the metal tendons are located further from the centroid towards the center of the circle than at the edge, and less concrete can also be used for the entire concrete roof structure. This results in an even lighter concrete roof structure.

According to a further embodiment, the cross-section from the center point of the circle to the edge of the concrete roof structure is substantially wedge-shaped. The wedge shape is advantageous because the height of the concrete roof structure decreases continuously. It is additionally advantageous that the concrete roof structure is predominantly defined by straight lines, which simplifies the construction of the concrete roof structure.

According to a preferred embodiment, a top surface of the concrete roof structure forms an upward angle of at most 10° with respect to a horizontal plane. "Upward angle" means that the concrete roof structure slopes upward at its top surface toward the center point of the circle. Preferably, the top surface forms an upward angle of at most 8° with respect to the horizontal plane, more preferably at most 6°, even more preferably at most 4°, and even more preferably at most 3°. This embodiment is advantageous because the concrete roof structure has only a very slight slope at its top surface, whereby technical infrastructure can be easily placed on top of the concrete roof structure and no walkways are needed on the concrete roof structure.

The top surface of the concrete roof structure preferably forms an upward angle of at least 1° with respect to the horizontal plane, more preferably at least 2°. This is advantageous to prevent rainwater from pooling on the concrete tank.

According to a preferred embodiment, a bottom surface of the concrete roof structure forms a downward angle of at least 2° with respect to a horizontal plane, preferably at least 3°, more preferably at least 4°, and even more preferably at least 5°. This embodiment is advantageous for obtaining more height near the center point of the circle, so that the metal tendons at the bottom surface can lie farther from the center of gravity, without the concrete roof structure thereby protruding higher at its top surface. A disadvantage of this embodiment is that an internal volume of the concrete tank slightly decreases.

This embodiment is particularly advantageous in combination with a previously described embodiment in which the top surface of the concrete roof structure forms an upward angle of at most 10° with respect to the horizontal plane.

According to a preferred embodiment, the concrete tank construction comprises a rectangular opening around a center point of the circle. The rectangular opening is, for example, an opening through which the optional agitator is placed into the concrete tank. At least a portion of the metal tendons are parallel to a first side of the rectangular opening. At least a portion of the metal tendons are parallel to a second side of the rectangular opening. The first side and the second side of the rectangular opening are perpendicular to each other. This embodiment is advantageous because grid patterns are thereby formed around the rectangular opening. The grid patterns form the greatest possible concentration of post-tensioned tendons around the rectangular opening, where the concrete roof structure is the heaviest and the stresses in the concrete roof structure are the highest.

This embodiment can advantageously be combined with a previously described embodiment, wherein at least a portion of the tendons form a grid pattern.

According to one embodiment, the concrete roof structure comprises a rectangular opening around a center point of the circle. The rectangular opening is, for example, an opening through which the optional agitator is placed into the concrete tank. The concrete roof structure comprises first additional metal tendons. The first additional metal tendons are located in the lower half of the concrete roof structure, similar to the previously discussed metal tendons. The first additional metal tendons form interrupted chords of the circle, wherein the interrupted chords are interrupted by the rectangular opening. The first additional metal tendons are post-tensioned. The post-tensioning is similar to that previously described for unbonded post-tensioning or bonded post-tensioning. The first additional metal tendons are preferably perpendicular to the rectangular opening. At least a portion of the first additional metal tendons are perpendicular to a first side of the rectangular opening. At least a portion of the first additional metal tendons are perpendicular to a second side of the rectangular opening. The first side and the second side of the rectangular opening are perpendicular to each other. The first additional metal tendons are advantageous for increasing a concentration of post-tensioned metal tendons around the rectangular opening, where the concrete roof structure is heaviest and the stresses in the concrete roof structure are highest.

This embodiment can advantageously be combined with a previously described embodiment, wherein at least a portion of the metal tendons are parallel to a first side and at least a portion of the metal tendons are parallel to a second side of the rectangular opening.

According to a preferred embodiment, the concrete roof structure is under compression at the top surface. This means that due to the post-tensioning of the metal tendons and optionally first additional metal tendons, the stresses in the lower half of the concrete roof structure are not fully compensated and the concrete roof structure sags minimally under its own weight. As a result, the bottom surface of the concrete roof structure is under tension, but the top surface of the concrete roof structure is under compression, which is advantageous for a gas-tight top surface of the concrete roof structure.

According to one embodiment, the cylindrical concrete side wall comprises an upright waterstop at an upper side. Preferably the waterstop is a waterstop made of polyvinyl chloride. The waterstop is cast into the cylindrical concrete side wall. The waterstop is also cast into the concrete roof structure. The waterstop is advantageous for a gas-tight connection of the concrete roof structure to the cylindrical concrete side wall. Preferably, a bituminous bearing material is applied on both sides of the waterstop on top of the cylindrical concrete side wall. This is also described as bitumen or roofing felt. This is advantageous for additional sealing between the cylindrical concrete side wall and the concrete top layer. Alternatively, a slip membrane is placed on both sides of the waterstop.

According to one embodiment, the metal tendons are tensioned with a force of at least 700 kN, preferably at least 800 kN, more preferably at least 900 kN, and even more preferably at least 1000 kN. This ensures that the concrete roof structure is sufficiently lifted.

According to one embodiment, the concrete roof structure comprises an upper reinforcement and a lower reinforcement. The upper reinforcement and the lower reinforcement are formed by reinforcement meshes and/or reinforcing bars. A position of the upper reinforcement and the lower reinforcement is preferably mutatis mutandis as described for the concrete floor slab. The reinforcing bars and/or the reinforcement meshes preferably have dimensions as described for the concrete floor slab. The upper reinforcement and the lower reinforcement are advantageous for supporting the dead weight of the concrete of the concrete roof structure and the optional agitator. As a result, the concrete roof structure needs to be lifted less by the metal tendons. The upper reinforcement and the lower reinforcement are preferably connected to each other near or above the cylindrical concrete side wall. The upper reinforcement and the lower reinforcement are preferably connected to each other near an opening for the optional agitator.

According to one embodiment, the metal tendons are located at a distance of at least 30 mm from a bottom surface of the concrete roof structure, more preferably at least 40 mm. The metal tendons are preferably located at a distance of at most 70 mm from the bottom surface of the concrete roof structure, more preferably at most 60 mm, even more preferably at most 55 mm, and even more preferably at most 50 mm. This embodiment is advantageous because the metal tendons are sufficiently covered by concrete to prevent damage and corrosion, while the metal tendons are located close enough to the bottom surface of the concrete roof structure to obtain a maximum effect from the post-tensioning of the metal tendons.

According to one embodiment, the concrete roof structure is post-tensioned by means of one or more loops of second additional metal tendons in the concrete roof structure. A second additional metal tendon is part of a single loop. A loop optionally comprises multiple tendons, preferably two tendons, more preferably three tendons.

Each loop is slidably positioned in a sheath. It is clear that the second additional metal tendon is slidably placed in the sheath. The sheath is as previously discussed for the metal tendon. The sheath is placed similarly as previously discussed. The sheath is placed in a formwork for the concrete roof structure before the casting of the concrete and is cast into the concrete. At a point where the loop is closed, an additional formwork is placed, into which no concrete is initially cast. At the point where the loop is closed, an anchor is placed. The anchor is as previously discussed for the metal tendon. Because no concrete is initially cast at the point where the loop is closed, the anchor is accessible and the loop can be tensioned after the concrete has cured. The anchor is preferably cast in concrete after the post-tensioning of the loop. The sheaths are not filled.

Alternatively, each loop is slidably placed in a duct in the concrete roof structure. At a point where the loop is closed, an additional formwork is placed, into which no concrete is initially cast. At the point where the loop is closed, an anchor for post-tensioning the loop is placed. After the concrete has cured, the loop is tensioned. The ducts in the concrete roof structure are filled with grout after the tensioning of each loop. The anchor is preferably encased in concrete after the post-tensioning of the metal tendon, so that the anchor is firmly integrated into the concrete roof structure.

Each loop substantially forms a circle around a center of the concrete roof structure. This means that a loop is circular but does not necessarily form a perfect circle, for example, because the loop deforms locally during the casting of the concrete or because the loop is not laid perfectly in a circle before the concrete is cast. Preferably, the loop lies close to an imaginary circle, wherein a center of the circle is the center of the concrete roof structure, and each point of the loop is at a distance of at most 50 cm from the imaginary circle, wherein the distance is measured along a radius of the circle. Preferably, the mentioned distance is at most 40 cm, more preferably at most 30 cm, even more preferably at most 20 cm, and even more preferably at most 10 cm. The one or more loops are particularly advantageous for compressing the concrete of the concrete roof structure with great force towards the center of the concrete roof structure.

When the concrete roof structure heats up or when there is overpressure from gas formed in the concrete tank, radial tensile forces normally arise in the concrete, leading to cracks. An agitator in the concrete tank is also usually suspended in or around the center of the concrete roof structure, again causing radial tensile forces. By compressing the concrete towards the center of the concrete roof structure, the tensile forces are compensated, and the concrete does not crack. As a result, the concrete roof structure remains gas-tight. This is not possible with only reinforcement meshes, as the bars of the reinforcement meshes are not radially oriented. Some of the bars of the reinforcement meshes will contribute little to absorbing the tensile forces. In addition, there must first be some stretch in the concrete before reinforcement meshes can absorb tensile forces, which means that cracks can already occur. The fact that the concrete roof structure remains gas-tight is particularly beneficial because the concrete roof structure has a very long lifespan of up to fifty years or more. Even when using a lining as an additional gas-tight barrier, a defect in the lining does not pose an immediate problem and a repair or renovation of the lining can be scheduled.

According to one embodiment, a protective layer for protecting the concrete and making the concrete tank gas-tight is applied to interior concrete surfaces of the concrete tank. Biomass and the formed gas contain sulfur, which affects the concrete interior surfaces and reduces the lifespan of the concrete tank. The protective layer forms an additional gas barrier. Non-limiting examples of possible protective layers include a rubber coating or rubber layer, an epoxy coating, an acrylate coating, and a plastic lining in the form of a film. The plastic lining in the form of a film is preferably at least 1 mm thick. The plastic lining in the form of a film is preferably at most 4 mm thick. A non-limiting list of suitable materials for the plastic lining includes polyethylene, polyvinyl chloride, polypropylene. The plastic lining preferably includes a flat side and a side with hooks. The side with hooks is particularly advantageous for good adhesion of the plastic lining in concrete when concrete is cast in situ. For example, the plastic lining is placed in formwork for the concrete side wall when the concrete side wall is cast in situ. Preferably, a plastic lining is applied as a protective layer on the concrete side wall and the floor slab. A plastic lining is more resistant to abrasive action by biomass than, for example, a rubber layer or a coating. For example, the plastic lining is placed in a formwork for the concrete roof structure during the in-situ casting of the concrete roof structure.

According to one embodiment, the concrete tank comprises a layer of roof insulation. The roof insulation layer is placed on an interior side of the concrete tank against the concrete roof structure. As a result, the roof of the concrete tank is accessible without the roof insulation being damaged. It is not necessary to install walkways on the roof of the concrete tank. The concrete roof structure can be walked on directly and the concrete tank can be constructed more simply.

The roof insulation layer is, for example, constructed with pre-formed boards made of extruded polystyrene, polyurethane, polyisocyanurate, or another suitable material. The panels are nailed, screwed, glued, or otherwise attached to the concrete roof structure.

Particularly advantageous about this embodiment is that, depending on a chosen insulation material, the layer of roof insulation already forms a first protection against sulfur in the methane gas formed in the concrete tank.

Preferably, the layer of roof insulation is formed in-situ. For example, the layer of roof insulation is a cured insulating concrete mortar. The insulating concrete mortar comprises expanded polystyrene granules. Such a cured concrete mortar is also known as EPS concrete, where EPS stands for Expanded Polystyrene. Insulating concrete mortar is advantageous because a high insulation value is achieved with a minimal layer thickness. The insulating concrete mortar is additionally advantageous because the roof insulation layer can be formed in place, for example in the formwork for casting the concrete roof structure. The primary function of the insulating concrete mortar is to insulate the concrete tank and it does not necessarily contribute to the structural strength of the concrete tank. This embodiment can advantageously be combined with an embodiment in which a plastic lining is installed under the roof insulation layer, preferably a plastic lining comprising protrusions. An alternative example of a layer of roof insulation that can be formed in-situ is a polyurethane insulating foam layer. Polyurethane insulating foam is very light and results in a high insulation rating. The use of polyurethane insulating foam results in similar advantages as a cured insulating concrete mortar. Polyurethane insulating foam has the disadvantage compared to insulating concrete mortar that favorable weather conditions are necessary to form the roof insulation layer in place. For example, it must not rain and it is preferably windless.

This embodiment can advantageously be combined with a previously described embodiment, wherein a protective layer for protecting the concrete and making the concrete tank gas-tight is applied to interior concrete surfaces of the concrete tank. If the layer of roof insulation is formed in-situ, the protective layer is preferably a plastic lining, more preferably a plastic lining with protrusions. The protrusions automatically engage in the layer of roof insulation during the formation of the layer of roof insulation. As a result, no additional steps are required, such as nailing, screwing, gluing, etc., to install the plastic lining under the roof insulation layer.

In a second aspect, the invention relates to a method for manufacturing a concrete tank for fermentation.

The method comprises the steps of:
- casting a concrete floor slab,
- casting a cylindrical concrete side wall,
- casting a concrete roof structure.

The concrete floor slab is preferably cast in place. The concrete floor slab is preferably reinforced using reinforcement meshes. Preferably, the floor slab comprises an upper reinforcement and a lower reinforcement. The concrete floor slab is cast directly onto a ground surface or onto a foundation. The foundation is a trench or pile foundation.

The concrete cylindrical side wall is preferably cast in place. The concrete cylindrical side wall is preferably cast layer by layer. The concrete side wall is preferably reinforced. The concrete side wall is reinforced using reinforcement meshes. Alternatively, the concrete side wall is reinforced with pre-tensioned or post-tensioned tendons, wherein the pre-tensioned or post-tensioned tendons are placed in circular loops in the cylindrical concrete side wall. Alternatively, the concrete cylindrical side wall is reinforced with a combination of at least two elements from a group consisting of reinforcement meshes, pre-tensioned tendons, and post-tensioned tendons. The concrete cylindrical side wall is preferably insulated.

The concrete roof structure is preferably reinforced.

The concrete roof structure is cast in place. For this, a temporary scaffolding is built inside the concrete tank to support a formwork for casting the concrete roof structure.

According to a preferred embodiment, the method comprises the additional steps of placing multiple metal tendons in the concrete roof structure and post-tensioning the multiple tendons after the curing of the concrete. The metal tendons are preferably steel tendons. The metal tendons form chords of a circle. The circle is concentric with a circular cross-section of the cylindrical concrete side wall. The meaning of concentric is as in the first aspect. The metal tendons are, viewed in a height direction, located in a lower half of the concrete roof structure.

Each metal tendon is slidably placed in a sheath. The sheath is preferably a plastic sheath that is placed in a formwork for the concrete roof structure before casting the concrete and which is cast into the concrete. Each metal tendon is attached at a first end to a fixed point in the concrete roof structure. At a point of a second opposite end of the metal tendon an additional formwork is placed, into which no concrete is cast at first. An anchor is placed at the point of the second end of the metal tendon. The anchor is suitable for post-tensioning the metal tendon. Preferably, after post-tensioning the metal tendon, the anchor is encased in concrete so that the anchor is securely integrated into the concrete roof structure. The sheaths are not filled. This form of post-tensioning is known as unbonded post-tensioning.

Alternatively, each metal tendon is slidably placed in a duct in the concrete roof structure. Each metal tendon is attached at a first end to a fixed point in the concrete roof structure. At a point of a second opposing end of the metal tendon, an additional formwork is placed, into which concrete is not initially cast. At the point of the second end of the metal tendon, an anchor for post-tensioning the metal tendon is placed. After the concrete has cured, the metal tendon is tensioned. After tensioning each metal tendon, the ducts in the concrete roof structure are filled with grout. Preferably, after post-tensioning the metal tendon, the anchor is encased in concrete so that the anchor is securely integrated into the concrete roof structure. This form of post-tensioning is known as bonded post-tensioning.

This method has the advantage, inter alia, that a concrete tank with a self-supporting and lighter concrete roof structure can be constructed with only a limited number of steps. It is additionally advantageous that the method also makes it possible to create the concrete roof structure flat or with only a very slight angle of inclination, which greatly simplifies the placement of technical infrastructure on the roof and makes walkways on the concrete roof structure superfluous. It is also advantageous that by carrying out the method, a concrete tank is obtained, wherein cracks in the concrete roof structure are avoided as much as possible, whereby the concrete roof structure remains gas-tight.

According to a preferred embodiment, at least a portion of the metal tendons is placed in a grid pattern. The grid pattern is preferably a rectangular grid pattern. The grid pattern is formed by the crossing of the metal tendons. This embodiment is advantageous because the greatest possible concentration of post-tensioned tendons is thereby obtained near the center of the circle, where the concrete roof structure is the heaviest and the stresses in the concrete roof structure are the highest.

According to a preferred embodiment, at least a portion of the metal tendons are placed as radii from a center of the circle. A radius is formed by one or more metal tendons. Multiple radii are formed by one or more tendons. This embodiment is advantageous because the greatest possible concentration of post-tensioned tendons is thereby obtained near the center of the circle, where the concrete roof structure is the heaviest and the stresses in the concrete roof structure are the highest.

This embodiment can advantageously be combined with a previously described embodiment, wherein at least a portion of the metal tendons are placed in a grid pattern.

According to a preferred embodiment, when casting the concrete roof structure, a concrete layer of decreasing height is cast from a center of the circle to an edge of the concrete roof structure. The height can decrease stepwise or continuously. Preferably, the height decreases continuously. Because a post-tensioning force remains the same over an entire length of a metal tendon, a greater perpendicular distance between the post-tensioning force and the center of gravity of the concrete roof structure is needed in the middle of the concrete roof structure to be able to compensate for the deflection of the concrete roof structure, than at an edge of the concrete roof structure. Because the concrete roof structure has a decreasing height from the center of the circle to the edge, the metal tendons are located further from the centroid towards the center of the circle than at the edge, and less concrete can also be used for the entire concrete roof structure. This results in an even lighter concrete roof structure.

According to a preferred embodiment, a protective layer is applied to the concrete interior surfaces of the concrete tank to protect the concrete and make the concrete tank gas-tight. Biomass and the formed gas contain sulfur, which affects the concrete interior surfaces and reduces the lifespan of the concrete tank. The protective layer forms an additional gas barrier. Non-limiting examples of possible protective layers include a rubber coating or rubber layer, an epoxy coating, an acrylate coating, and a plastic lining in the form of a film.

A person skilled in the art will appreciate that a method according to the second aspect is preferably carried out for manufacturing a concrete tank according to the first aspect and that a concrete tank according to the first aspect is preferably manufactured by carrying out a method according to the second aspect. Each feature described in this document, both above and below, can therefore relate to any of the three aspects of the present invention.

In a third aspect, the invention relates to a use of a concrete tank according to a first aspect for pre-acidification and/or biomass fermentation.

This use results in advantageous pre-acidification (hydrolysis) and/or fermentation of biomass in a concrete tank, wherein because of the concrete roof structure of the concrete tank, no or only very minimal methane gas escapes from the concrete tank. Methane gas is harmful to the climate. By avoiding cracks in the concrete roof structure, a higher gas yield from the fermentation can also be obtained.

In what follows, the invention is described by way of non-limiting figures illustrating the invention, and which are not intended to and should not be interpreted as limiting the scope of the invention.

### DESCRIPTION OF THE FIGURES

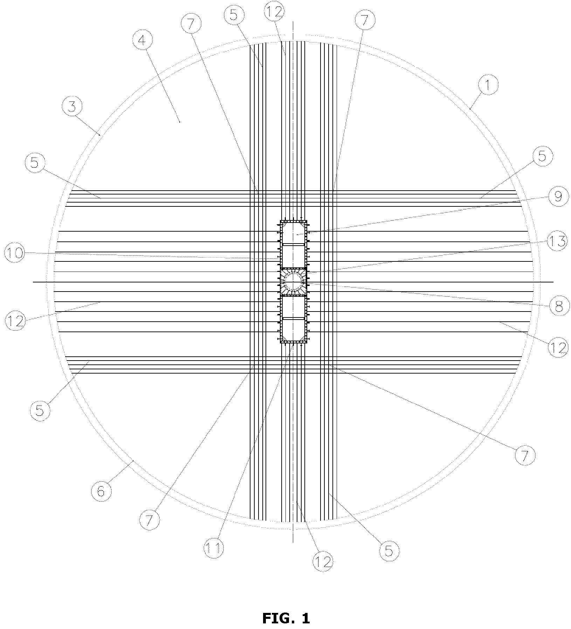

**Figure 1** shows a top view of a concrete tank according to an embodiment of the present invention.

The concrete tank (1) comprises a concrete floor slab (2), a cylindrical concrete side wall (3), and a concrete roof structure (4). The concrete floor slab (2) is not visible in Figure 1. The concrete roof structure (4) is a self-supporting concrete structure. The concrete roof structure (4) rests on top of the cylindrical concrete side wall (3) and has a diameter that is equal to an outer diameter of the cylindrical concrete side wall (3). The concrete roof structure (4) conceals underlying structures of the concrete tank (1). These underlying structures are nevertheless shown in Figure 1 for illustration. The concrete roof structure (4) is post-tensioned using multiple metal tendons (5). The metal tendons (5) are placed in sheaths. The sheaths are not shown in Figure 1. The metal tendons (5) form chords of a circle (6). The chords connect points on a circumference of the circle (6). The circle (6) is concentric with a circular cross-section of the cylindrical concrete side wall (3). In this embodiment, the circle (6) has a radius that is equal to a radius of the cylindrical concrete side wall (3), wherein the radius of the cylindrical concrete side wall (3) is measured on an interior side of the concrete tank (1). The metal tendons (5) form a grid pattern (7). The grid pattern (7) is a rectangular grid pattern (7). The metal tendons (5) cross each other at a distance from a center point of the circle (6) that is greater than 40% of a radius of the circle (6). The concrete tank (1) in this embodiment comprises an agitator (8). The agitator (8) is optional. The concrete roof structure (4) comprises a rectangular opening (9) around the center point of the circle (6). The agitator (8) is placed through the rectangular opening (9) into the concrete tank (1). Some of the metal tendons (5) are parallel to a first side (10) of the rectangular opening (9). Some of the metal tendons (5) are parallel to a second side (11) of the rectangular opening (9). The first side (10) and the second side (11) are perpendicular to each other. The concrete roof structure (4) comprises first additional metal tendons (12). The first additional metal tendons (12) form interrupted chords of the circle (6). The interrupted chords are interrupted by the rectangular opening (9). The first additional metal tendons (12) are post-tensioned. The first additional metal tendons (12) are also placed in sheaths. These sheaths are also not shown in Figure 1. The first additional metal tendons (12) are transverse to the rectangular opening (9). Some of the first additional metal tendons (12) are perpendicular to the first side (10) of the rectangular opening (9). Some of the first additional metal tendons (12) are perpendicular to the second side (11) of the rectangular opening (9).

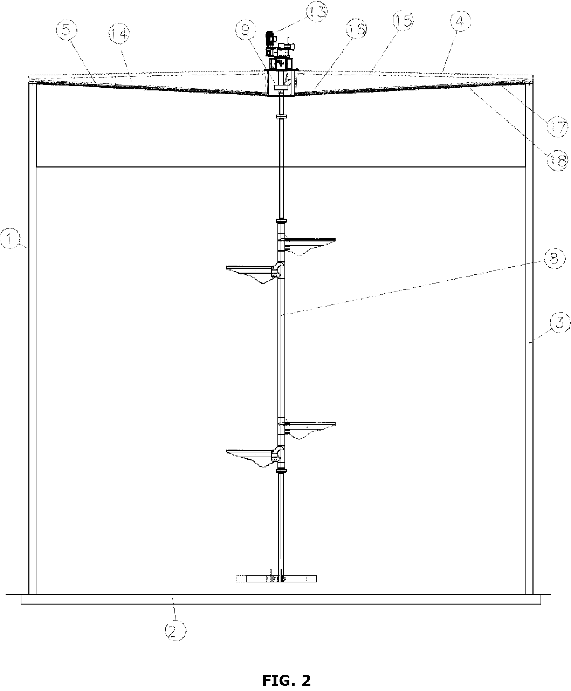

**Figure 2** shows a cross-section in a vertical plane of a concrete tank according to an embodiment of the present invention.

The cross-section is a view of a concrete tank (1) as in Figure 1. The concrete floor slab (2) was cast in place. It is clearly visible how an agitator (8) is suspended centrally in the concrete tank (1) from the concrete roof structure (4). On top of the concrete roof structure (4), a motor (13) for driving the agitator (8) is placed. In Figure 2, it can be seen how the metal tendons (5) are located in a lower half of the concrete roof structure (4). The metal tendons (5) run parallel to a bottom surface of the concrete roof structure (4). It can also be seen that the concrete roof structure (4) has a decreasing height from the center point of the circle (6) to an edge of the concrete roof structure (4). The height decreases continuously. The cross-section of the concrete roof structure (4) is substantially wedge-shaped from the center point of the circle (6) to the edge of the concrete roof structure (4). A top surface of the concrete roof structure (4) forms an upward angle of 2° with respect to a horizontal plane. A bottom surface of the concrete roof structure (4) forms a downward angle of 3° with respect to the horizontal plane. In this embodiment, the concrete roof structure (4) is shaped like a disk. The concrete roof structure (4) comprises an upper reinforcement (15) and a lower reinforcement (16) with reinforcing bars (14). The concrete tank (1) comprises a layer of roof insulation (17). The layer of roof insulation (17) is placed on an interior side of the concrete tank (1) against the concrete roof structure (4). In this embodiment, the layer of roof insulation (17) is a cured insulating concrete mortar, more specifically EPS concrete. Under the layer of roof insulation (17), a protective plastic lining (18) with protrusions is provided. The protrusions engage in the layer of roof insulation (17). The protective plastic lining (18) is also provided in the rectangular opening (9).

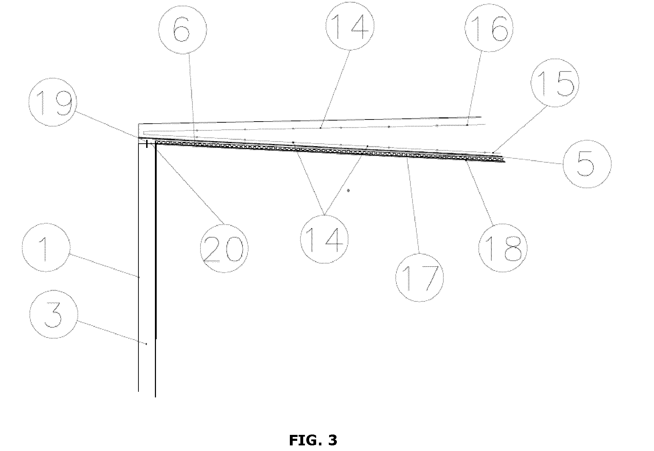

**Figure 3** shows a detailed cross-section in a vertical plane of a concrete tank according to an embodiment of the present invention.

Figure 3 shows in more detail a corner formed by the concrete roof structure (4) and the cylindrical concrete side wall (3) of a concrete tank (1). The concrete tank (1) corresponds to the concrete tank (1) from Figure 1 and Figure 2. The metal tendons (5), the upper reinforcement (15), the lower reinforcement (16), the layer of roof insulation (17), and the protective plastic lining (18) are more clearly visible in Figure 3. A protective layer is applied to the concrete interior surfaces of the concrete tank (1) to protect the concrete and make the concrete tank gas-tight. Under the concrete roof structure, this is the protective plastic lining (18). The cylindrical concrete side wall (3) comprises an upright waterstop (19) at an upper side. The waterstop (19) is cast into the cylindrical concrete side wall (3). The waterstop (19) is also cast into the concrete roof structure (4). On both sides of the waterstop (19), a slip membrane (20) is placed on the top surface of the cylindrical concrete side wall (3).

The numbered elements in the figures are:
1. Concrete tank
2. Concrete floor slab
3. Cylindrical concrete side wall
4. Concrete roof structure
5. Metal tendons
6. Circle
7. Grid pattern
8. Agitator
9. Rectangular opening for agitator
10. First side of the rectangular opening
11. Second side of the rectangular opening
12. First additional metal tendons
13. Motor
14. Reinforcing bar
15. Upper reinforcement
16. Lower reinforcement
17. Roof insulation
18. Plastic lining
19. Waterstop
20. Slip membrane

## Claims

1. A concrete tank for fermentation comprising a concrete floor slab, a cylindrical concrete side wall, and a concrete roof structure, **characterized in that** the concrete roof structure is a self-supporting concrete structure, wherein the concrete roof structure is post-tensioned by means of a plurality of metal tendons in the concrete roof structure, wherein the metal tendons form chords of a circle, wherein the circle is concentric with a circular cross-section of the cylindrical concrete side wall and wherein the metal tendons, as viewed in a height direction, are located in a lower half of the concrete roof structure.

2. The concrete tank according to claim 1, **characterized in that** at least a portion of the metal tendons form a grid pattern.

3. The concrete tank according to claim 1 or 2, **characterized in that** at least a portion of the metal tendons are arranged as radii extending from the center of the circle.

4. The concrete tank according to any one of the preceding claims 1-3, **characterized in that**, as viewed in a vertical plane through the center of the circle, the concrete roof structure has a cross-section with a height that decreases from the center of the circle toward an edge of the concrete roof structure.

5. The concrete tank according to claim 4, **characterized in that** the cross-section is substantially wedge-shaped from the center of the circle to the edge of the concrete roof structure.

6. The concrete tank according to any one of the preceding claims 1-5, **characterized in that** a top surface of the concrete roof structure forms an upward angle of at most 10° with respect to a horizontal plane.

7. The concrete tank according to any one of the preceding claims 1-6, **characterized in that** a bottom surface of the concrete roof structure forms a downward angle of at least 2° with respect to a horizontal plane.

8. The concrete tank according to any one of the preceding claims 1-7, **characterized in that** the concrete tank structure comprises a rectangular opening around the center of the circle, wherein at least a portion of the metal tendons are parallel to a first side of the rectangular opening, wherein at least a portion of the metal tendons are parallel to a second side of the rectangular opening, and wherein the first side and the second side are perpendicular to each other.

9. The concrete tank according to any one of the preceding claims 1-8, **characterized in that** the concrete roof structure is in compression at its top surface.

10. A method for manufacturing a concrete tank for fermentation comprising the steps of:
- casting a concrete floor slab;
- casting a cylindrical concrete side wall;
- casting a concrete roof structure;
**characterized in that** the method comprises the additional steps of placing a plurality of metal tendons in the concrete roof structure, wherein the metal tendons form chords of a circle, wherein the circle is concentric with a circular cross-section of the cylindrical concrete side wall and wherein the metal tendons, as viewed in a height direction, are placed in a lower half of the concrete roof structure, and post-tensioning the plurality of tendons after the curing of the concrete.

11. The method according to claim 10, **characterized in that** at least a portion of the metal tendons are placed in a grid pattern.

12. The method according to claim 10 or 11, **characterized in that** at least a portion of the metal tendons are placed as radii from a center of the circle.

13. The method according to claim 10, 11, or 12, **characterized in that**, during the casting of the concrete roof structure, a concrete layer of decreasing height is cast from a center of the circle toward an edge of the concrete roof structure.

14. The method according to any one of claims 10-13, **characterized in that** a protective layer is applied to the interior concrete surfaces of the concrete tank, to protect the concrete and ensure the tank is gas-tight.

15. Use of a concrete tank according to any one of claims 1-9 for pre-acidification and/or biomass fermentation.
